# EUROPÄISCHE PATENTSCHRIFT

(11) **EP 1 148 071 B1**
(45) Veröffentlichungstag und Bekanntmachung des Hinweises auf die Patenterteilung: **16.06.2004**
(21) Anmeldenummer: 01107895.3
(22) Anmeldetag: 11.04.2001
(51) Int. Cl.: C08F 30/02, A61K 6/00

(54) **Hydrolysestabile und polymerisierbare Acrylphosphonsäuremonoester**
Hydrolysis-stable and polymerisable acrylphosphonic acid mono esters
Mono esters de l'acide acrylphosphonique stables à l'hydrolyse et polymérisables

(30) Priorität: 17.04.2000 DE 10018969
(43) Veröffentlichungstag der Anmeldung: 24.10.2001
(73) Patentinhaber: Ivoclar Vivadent AG, 9494 Schaan (LI)
(72) Erfinder: Moszner, Norbert, 9492 Eschen (LI); Rheinberger, Volker, 9490 Vaduz (LI); Zeuner, Frank, 9490 Vaduz (LI)
(74) Vertreter: UEXKÜLL & STOLBERG

(56) Entgegenhaltungen:
- DE-A- 19 746 708
- US-A- 4 499 251
- ZEUNER F ET AL: "SYNTHESIS AND DENTAL ASPECTS OF ACRYLIC PHOSPHORIC AND PHOSPHONIC ACIDS" , PHOSPHORUS, SULFUR AND SILICON AND THE RELATED ELEMENTS, GORDON AND BREACH SCIENCE PUBLISHERS, AMSTERDAM, GB, VOL. 144-146, PAGE(S) 133-136 XP008000438 ISSN: 1042-6507 * das ganze Dokument *

## Beschreibung

Die vorliegende Erfindung betrifft polymerisierbare Acrylphosphonsäuremonoester, die eine hohe Hydrolysestabilität aufweisen und sich insbesondere zur Herstellung oder als Bestandteile von Polymeren, Adhäsiven oder anderen Materialien und hauptsächlich von Dentalmaterialien eignen.

Polymerisierbare Phosphonsäuren sind vor allem als Comonomere von polymer-chemischer Bedeutung. Sie gestatten die Herstellung von organischen Polymeren mit hoher thermischer Stabilität, guten Hafteigenschaften, schwerer Entflammbarkeit und guter Löslichkeit in polaren Lösungsmitteln. Für diesen Zweck sind zahlreiche monomere Phosphonsäuren mit polymerisierbaren Vinyl-, Dienyl-, Allyl- oder Styrylgruppen synthetisiert und polymerisiert worden. Eine Übersicht zu Phosphonsäuren gibt Houben-Weyl, Methoden oder Organischen Chemie, Band E 20 (2. Teil), Georg Thieme Verlag, Stuttgart-New York 1987, S. 1300 ff. Beispiele für solche konventionellen polymerisierbaren Phosphonsäuren sind Vinylphosphonsäure, Allylbenzolphosphonsäure, α-Aminoallylphosphonsäure, Phenylethenphosphonsäure, 1,3-Butadien- oder Isoprenphosphonsäure, 4-Vinylbenzolphosphonsäure oder 2-(4-Vinylphenyl)-ethanphosphonsäure.

Phosphonsäuren, bei denen die C=C-Gruppierung direkt oder über ein Sauerstoffatom an das Phosphoratom gebunden ist, wie z.B. Vinylphosphonsäure oder Ethylphosphonsäuremonovinylester, zeigen allerdings nur eine mäßige Neigung zur Homopolymerisation, so daß nur Homopolymere mit einer geringen Molmasse zugänglich sind.

Hochmolekulare Polymerisate können demgegenüber von (Meth)acrylphosphonsäuren oder -estern erhalten werden, bei denen die (Meth)acrylgruppe nicht direkt sondern über eine hydrolysestabile Spacergruppe an den Phosphor gebunden ist. Derartige (Meth)acrylphosphonsäure-Derivate werden beispielsweise in der DE-B-27 11 234 beschrieben.

Die DE-A-32 10 775 offenbart 2-Acrylamido-2-methyl-propanphosphonsäure mit der Formel CH₂=CH-CONH-C(CH₃)₂-CH₂-P(=O) (OH)₂ sowie deren Verwendung zur Herstellung von Copolymerisaten.

Die DE-A-33 13 819 und die JP 62-63314 (Chem. Abstr. 107 (1987), 41318f) offenbaren Methacrylsäure-(2-phosphono-1,1-dimethylethylamin) der Formel CH₂=C(CH₃)-CONH-C(CH₃)₂-CH₂-P(=O)(OH)₂.

Gemäß EP-B-0 089 654 und US-A-4 650 591 soll sich Acrylsäure-(2-phosphono-1,1-dimethylethylamin), auch als 2-Acrylamido-2-methylpropanphosphonsäure bezeichnet, in Form ihrer Homo- oder Copolymeren als Korrosionsinhibitor eignen.

Die DD-A-273 846 offenbart Haftvermittler auf der Basis von N-Acyl-aminomethan-bisphosphonsäurederivaten.

Diese bekannten (Meth)acrylphosphonsäure-Derivate sind in wäßriger Lösung nicht stabil. Vielmehr findet bei ihnen eine hydrolytische Abspaltung der (Meth)acrylgruppe statt, die durch dissoziierte Protonen der Phosphonsäuregruppe sogar noch katalysiert und damit beschleunigt wird.

Bei einer ganzen Reihe von Anwendungen polymerisierbarer Phosphonsäuren ist jedoch der Einsatz von wäßrigen Lösungen von Vorteil oder zwingend notwendig. Dies ist z.B. bei der Herstellung von niedrigviskosen Adhäsiven, die frei von organischen Lösungsmitteln sind, oder bei dentalen Adhäsiven der Fall, die nur in wäßriger Form zu einer optimalen Benetzung der feuchten Dentinoberfläche führen.

Die DE 197 46 708 A1 offenbart polymerisierbare Acrylphosphonsäuren, die in wäßriger Lösung hydrolysestabil sind, über gute Hafteigenschaften verfügen, mit herkömmlichen radikalischen Initiatoren polymerisiert werden können und sich daher als Bestandteil insbesondere von Adhäsiven, Formkörpern, Zementen oder Kompositen und vor allem von Dentalmaterialien eignen. Die Acrylphosphonsäuren zeigen in Form ihrer Carbonsäureester eine gute Löslichkeit in Wasser und polaren organischen Lösungsmitteln während sie in Form der Carbonsäuren zwar gut in Wasser aber kaum in organischen Lösungsmitteln löslich sind. Das unterschiedliche Lösungsverhalten von Ester und Säure kann bei wasserhaltigen Materialien nachteilig sein. Die Hydrolyse der Carbonsäureester zur freien Carbonsäure unter Abspaltung von Alkohol kann die Löslichkeit der Monomeren signifikant verändern und so zur partiellen oder vollständigen Ausfällung der Phosphonsäurekomponente führen und damit die Eigenschaften des Materials beeinflussen.

Aufgabe der Erfindung ist die Bereitstellung von hydrolysestabilen polymerisationsfähigen Acrylphosphonsäure-Derivaten, die gut in Wasser und polaren organischen Lösungsmitteln löslich sind und deren Lösungsverhalten nicht durch Hydrolyse verändert wird.

Diese Aufgabe konnte überraschend durch Acrylphosphonsäureester der folgenden allgemeinen Formel (I) gelöst werden 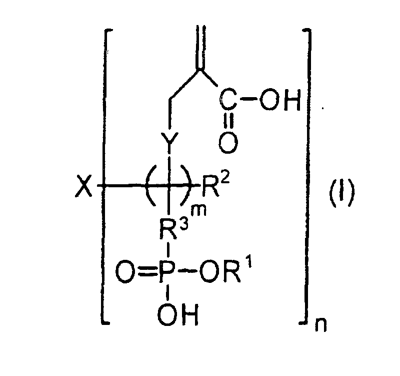 in der R¹, R², R³, X, Y, m und n, unabhängig voneinander die folgenden Bedeutungen haben:
- R¹ =: ein geradkettiger oder verzweigter C₁- bis C₂₀-Alkyl- oder C₆- bis C₁₄-Aryl-Rest;
- R² =: Wasserstoff, ein geradkettiger oder verzweigter C₁- bis C₅-Alkyl- oder Phenylrest;
- R³ =: ein geradkettiger oder verzweigter C₁- bis C₈-Alkylenrest, Phenylen oder entfällt;
- Y =: Sauerstoff, C₁- bis C₈-Alkylen oder entfällt;
- m =: 0 oder 1;
- n =: 1 oder 2;
mit der Maßgabe, daß nicht gleichzeitig Y = O, m = 0 und R³ = entfällt bedeuten können und
mit der weiteren Maßgabe daß
für m = 1 und n = 1
- X =: Wasserstoff oder ein geradkettiger oder verzweigter C₁-bis C₅-Alkylrest oder ein C₆- bis C₁₄-Arylrest;
für m = 1 und n = 2
- X =: ein geradkettiger oder verzweigter C₁- bis C₁₀-Alkylen-, C₆- bis C₁₀-Arylen-, C₇- bis C₂₀-Arylalkylenrest oder eine chemische Bindung, die zwei Reste mit der in Klammern stehenden Struktur der Formel (I) miteinander verbindet.

Die einzelnen Alkyl- und Alkylenreste können dabei geradkettig, verzweigt oder cyclisch sein. Außerdem können die einzelnen Alkyl-, Aryl-, Alkylen-, Arylen-, Phenyl-, Phenylen- und Arylenalkylenreste einen oder mehrere, vorzugsweise 1 bis 2 Substituenten, wie Cl, Br, CH₃O, COOH, CN oder vorzugsweise OH tragen.

R¹ ist vorzugsweise unsubstituiert oder durch eine oder mehrere OH-Gruppen, vorzugsweise 1 oder 2 OH-Gruppen substituiert. Die übrigen Reste sind vorzugsweise nicht substituiert.

Weiter existieren für die oben angegebenen Variablen der Formel (I) bevorzugte Definitionen, die, sofern nicht anders angegeben, unabhängig voneinander gewählt werden können und wie folgt sind:
- R¹ =: ein geradkettiger oder verzweigter C₁- bis C₁₀-Alkyl- oder Phenylrest;
- R² =: Wasserstoff oder ein geradkettiger oder verzweigter C₁- bis C₃-Alkylrest;
- R³ =: ein geradkettiger oder verzweigter C₁- bis C₄-Alkylenrest, Phenylen oder entfällt;
- Y =: Sauerstoff oder entfällt; und
- X =: Wasserstoff oder ein geradkettiger oder verzweigter C₁- bis C₃-Alkylrest (für m = 1 und n = 1); oder
- X =: ein geradkettiger oder verzweigter C₁- bis C₆-Alkylenrest, Phenylen oder eine chemische Bindung, die zwei Reste mit der in Klammern stehenden Struktur der Formel (I) miteinander verbindet (für m = 1 und n = 2).

Besonders bevorzugte und ebenfalls unabhängig voneinander wählbare Bedeutungen sind:
- R¹ =: ein geradkettiger oder verzweigter C₁- bis C₄-Alkylrest, der unsubstituiert oder durch eine OH-Gruppen substituiert sein kann;
- R² =: Wasserstoff oder ein geradkettiger oder verzweigter C₁- bis C₃-Alkylrest;
- R³ =: ein geradkettiger oder verzweigter C₁- bis C₄-Alkylenrest, Phenylen oder entfällt;
- Y =: Sauerstoff oder entfällt.

Weiterhin sind Acrylphosphonsäuremonoester besonders bevorzugt, bei denen R¹, R², R³, Y, und n die oben angegebenen Bedeutungen haben und (i) m = 0 ist oder (ii) m = 1, n = 2 und
- X =: Phenylen oder eine chemische Bindung, die zwei Reste mit der in Klammern stehenden Struktur der Formel (I) miteinander verbindet.

Bevorzugte Verbindungen sind demgemäß solche, bei denen mindestens eine, besonders bevorzugt alle der Variablen der Formel (I) die vorstehend beschriebenen bevorzugten Definition aufweisen, wobei die Formel (I) alle durch die genannten Substituenten möglichen Stereoisomere und ihre Mischungen, wie Racemate, einschließt.

Wenn m gleich 0 ist, entfallen die Reste X und R².

Die erfindungsgemäßen Acrylphosphonsäuremonoester (APME) der Formel (I) lassen sich durch partielle Hydrolyse entsprechender Acrylphosphonsäureester APE herstellen. Hierzu kann verdünnte Natronlauge eingesetzt werden, eine Silylierung beispielsweise mit Trialkylsilanen ist nicht erforderlich, so daß die Acrylphosphonsäuremonoester einfacher und kostengünstiger zugänglich sind als vergleichbare Phosphonsäuren.

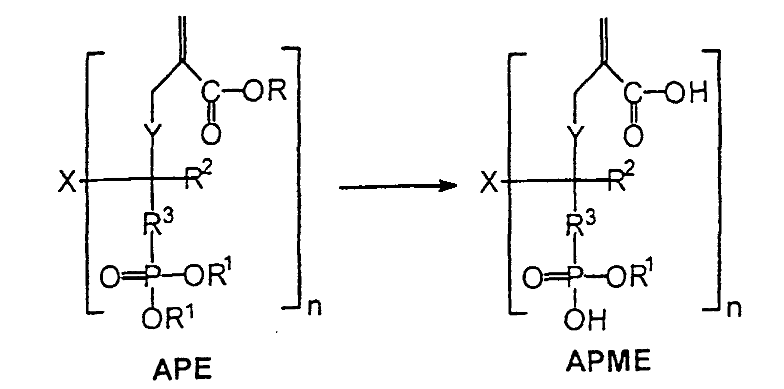

Konkret ergibt die Umsetzung von 2-[4-Dimethoxyphosphoryl-2-oxabutyl]-acrylsäureethylester mit Natronlauge den entsprechenden Phosphonsäuremonoester (2-[4-Hydroxymethoxyphosphoryl-2-oxabutyl)-acrylsäure):

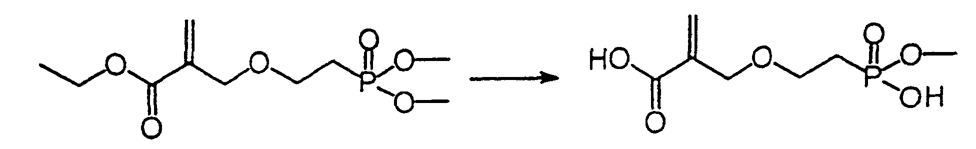

Die hierzu eingesetzten Acrylphosphonsäureester (APE) sind beispielsweise durch Umsetzung von α-Halogenmethylacrylsäureestern (HMAE; U = Halogen, vorzugsweise Cl oder Br) mit monooder difunktionellen Phosphonsäureestern (PE) unter Anwendung der aus der organischen Chemie bekannten Methoden für die Knüpfung von C-C, C-O oder C-S-Bindungen erhältlich (vgl. C. Weygand, G. Hilgetag, Organisch-chemische Experimentierkunst, Johann Ambrosius Bart Verlag, Leipzig 1970, Seiten 963 ff., 362 ff., 657 ff.; N. Moszner, F. Zeuner, U.K. Fischer, V. Rheinberger, Macromol. Chem. Phys. 200 (1999) 1062. Diese Umsetzung kann durch die folgende allgemeine Reaktionsgleichung veranschaulicht werden:

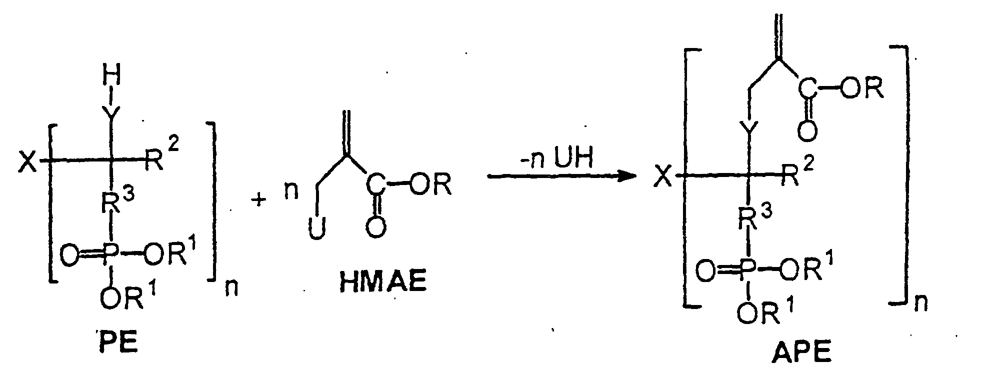

Konkret führt die Umsetzung von α-Chlormethylacrylsäureethylester mit 2-Hydroxyethylphosphonsäuredimethylester zum 2-[4-Dimethoxyphosphoryl-2-oxa-butyl)-acrylsäureethylester:

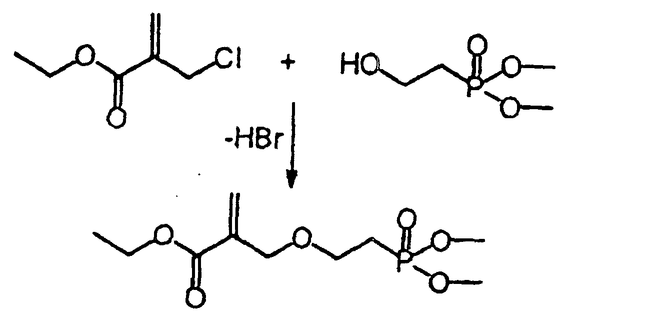

Eine weitere Herstellungsmöglichkeit besteht in der Umsetzung entsprechender Acrylphosphonsäure (APA) mit Epoxiden unter Bildung von OH-substituierten Acrylphosphonsäuremonoestern OH-APME. Die Reaktion kann analog zu B. Costisella, H. Gross, J. Prakt. Chem. 317 (1975) 798 durchgeführt werden.

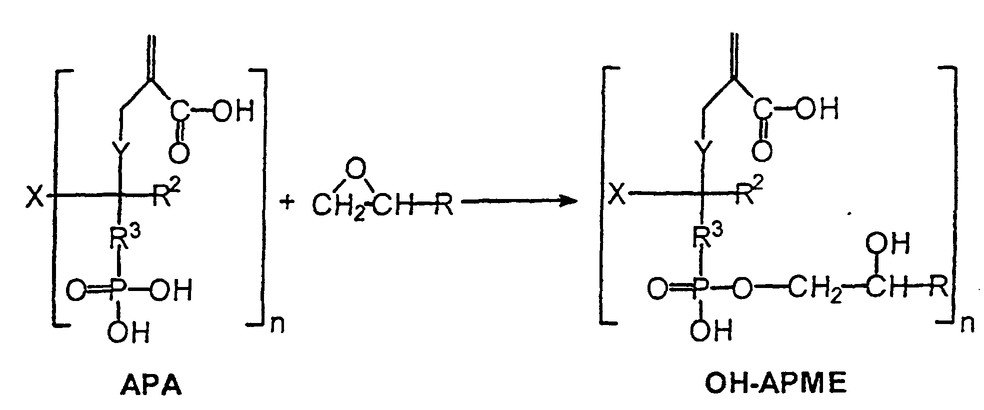

Konkret ergibt die Umsetzung von 2-[4-Dihydroxyphosphoryl-2-oxabutyl]-acrylsäure mit Propylenoxid den entsprechenden Phosphonsäuremonoester (2-{4-[Hydroxy-(2-hydroxypropoxy)]-phosphoryl-2-oxa-butyl}-acrylsäure):

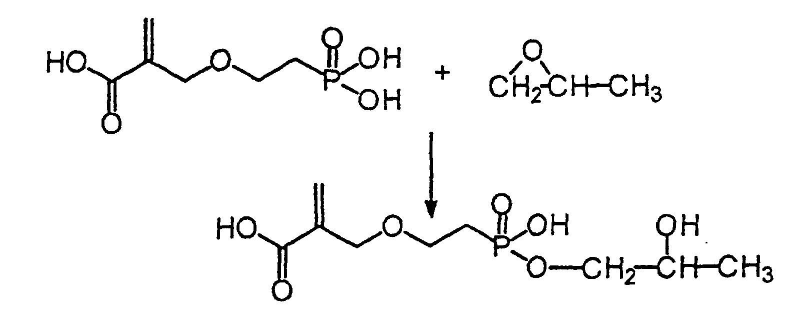

Beispiele für die erfindungsgemäßen Acrylphosphonsäuren der Formel (I) sind u.a.:

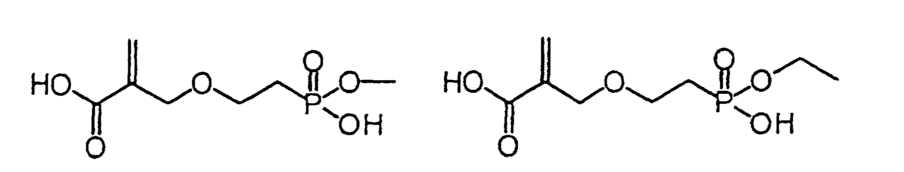

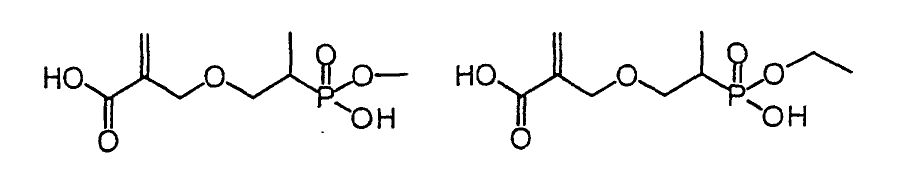

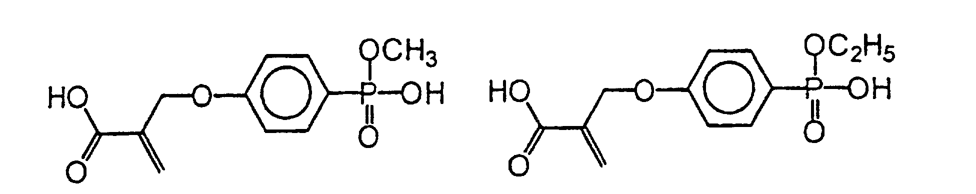

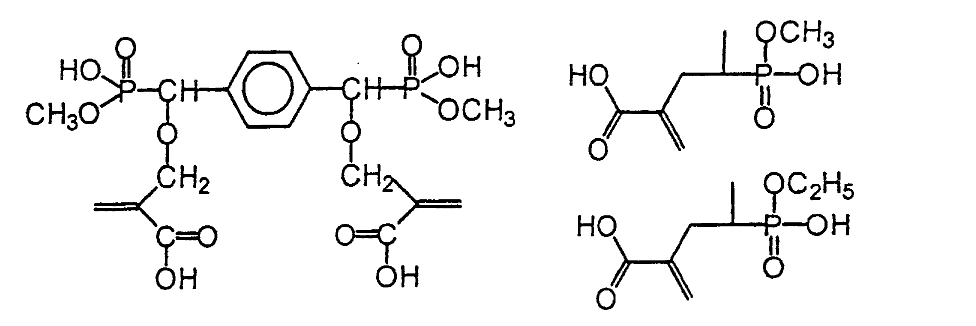

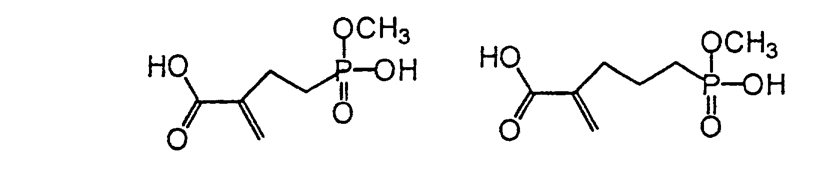

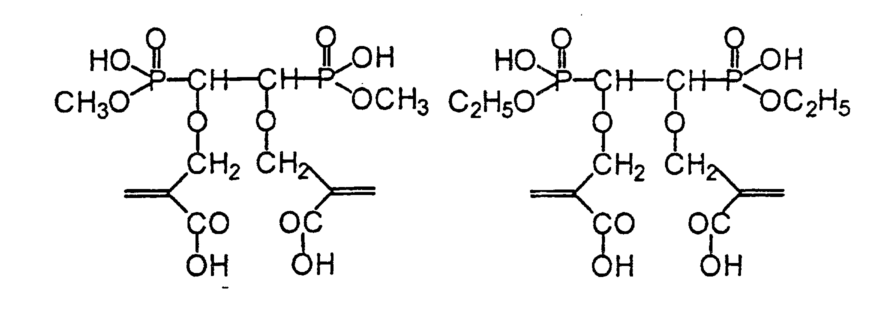

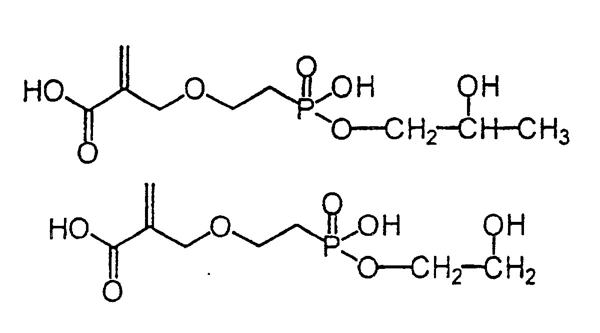

Die erfindungsgemäßen Acrylphosphonsäuremonoester sind im Vergleich zu den entsprechenden Acrylphosphonsäuren wesentlich besser in Mischungen aus polaren organischen Lösungsmitteln, wie Methanol, Ethanol, Isopropanol, Methylethylketon, Aceton, Essigsäureethylester, Dimethylformamid oder Dimethylsulfoxid, und Wasser löslich, weisen aber auch in den polaren organischen Lösungsmitteln selbst eine hohe Löslichkeit auf.

Um eine ausreichende Schmelz- und Dentinhaftung von Dentalwerkstoffen zu erzielen, werden nach der Präparation beispielsweise einer Kavität üblicherweise die Schmelzränder und das Dentin mit 35 bis 40%-iger Phosphorsäure für jeweils etwa 20 bis 30 Sekunden angeätzt. Die erfindungsgemäßen Acrylphosphonsäuremonoester weisen im Vergleich zu den entsprechenden Acrylphosphonsäuren überraschenderweise eine deutlich höhere Acidität und damit eine stärkere selbstätzende Wirkung auf Schmelz und Dentin auf, so daß auf ein zusätzliches Anätzen von Schmelz und Dentin verzichtet werden kann.

Aufgrund des Vorliegens von polymerisierbaren Gruppen eignen sich die erfindungsgemäßen Acrylphosphonsäureester als Ausgangsmaterialien für die Herstellung von Polymeren und Copolymeren. Dabei lassen sie sich mit den bekannten Methoden der radikalischen Polymerisation homopolymerisieren oder z.B. mit geeigneten Comonomeren copolymerisieren.

Zur Durchführung der Polymerisation können die bekannten radikalischen Initiatoren (vgl. Encyclopedia of Polymer Science and Engineering, Vol. 13, Wiley-Interscience Publisher, New York 1988, 754 ff.) eingesetzt werden. Es eignen sich besonders Azoverbindungen, wie Azobis(isobutyronitril) (AIBN) oder Azobis(4-cyanvaleriansäure) oder Peroxide, wie Dibenzoylperoxid, Dilauroylperoxid, tert.-Butylperoctoat, tert.-Butylperbenzoat oder Di-(tert.-butyl)-peroxid.

Als Initiatoren für die Heißhärtung eignen sich auch Benzpinakol und 2,2'-Dialkylbenzpinakole.

Weiterhin können auch Photoinitiatoren (vgl. J.P. Fouassier, J.F. Rabek (Hrsg.), Radiation Curing in Polymer Science and Technology, Vol. II, Elsevier Applied Science, London und New York 1993) für die Polymerisation mit UV-Licht oder Licht sichtbarer Wellenlängen, wie Benzoinether, Dialkylbenzilketale, Dialkoxyacetophenone, Acylphosphinoxide, α-Diketone, wie 9,10-Phenanthrenchinon, Diacetyl, Furil, Anisil, 4,4'-Dichlorbenzil und 4,4'-Dialkoxybenzil, und Campherchinon, verwendet werden.

Die erfindungsgemäßen Acrylphosphonsäuremonoester können insbesondere als Bestandteil von Adhäsiven, Zementen, Kompositen und Formkörpern sowie bevorzugt von Dentalmaterialien verwendet werden. Die erfindungsgemäßen Acrylphosphonsäuremonoester können dabei auch in polymerisierter oder teilweise polymerisierter Form, d.h. in Form von Polymeren wie Homo- oder Copolymeren, eingesetzt werden, beispielsweise als Komponente von Glasionomerzementen.

Die erfindungsgemäßen Acrylphosphonsäuremonoester lassen sich allein oder in Mischung mit herkömmlichen radikalisch polymerisierbaren Comonomeren, insbesondere mit difunktionellen Vernetzermonomeren polymerisieren. Für die Herstellung von Adhäsiven oder Dentalmaterialien eignen sich vor allem vernetzende bi- oder mehrfunktionelle Acrylate oder Methacrylate, wie z.B. Bisphenol-A-di(meth)acrylat, Bis-GMA (das Additionsprodukt von Methacrylsäure und Bisphenol-A-diglycidylether), UDMA (das Additionsprodukt von Hydroxyethylmethacrylat und 2, 2, 4 -Trimethylhexamethylendiisocyanat), Di-, Tri- oder Tetraethylenglykoldi(meth)acrylat, Trimethylolpropantri(meth)acrylat und Pentaerythrittetra(meth)acrylat. Es eignen sich ebenfalls die durch Veresterung von (Meth)acrylsäure mit den entsprechenden Diolen zugänglichen Verbindungen Butandioldi(meth)acrylat, 1,10-Decandioldi(meth)-acrylat und 1,12-Dodecandioldi(meth)acrylat.

Die erfindungsgemäßen Acrylphosphonsäuremonoester können in freier Form oder in Form ihrer Salze, d.h. als Phosphonatester, eingesetzt werden, wobei im Fall der Salze als Gegenionen vorzugsweise Alkalimetallionen, insbesondere Natrium- und Lithiumionen, sowie organische Ammoniumionen dienen, insbesondere solche, die sich von Aminbeschleunigern, wie N,N-Dihydroxyethylp-toluidin, N,N-Bis-(2-hydroxy-3-methacryloxypropyl-3,5-xylidin oder 4-(Dimethylamino)-benzoesäure-2-ethyl-hexylester, ableiten. Aminbeschleuniger werden im Dentalbereich als Komponente beispielsweise von Photoinitiatorsystemen eingesetzt. Es handelt sich im allgemeinen um tert. Amine, die als H-Donatoren wirken können und damit die Radikalbildung beschleunigen (vgl. L.A. Linden, "Photocuring of Polymeric Dental Materials and Plastic Composite Resins" in Radiation Curing in Polymer Science and Technology, Vol. IV, J.P. Fouassier, J.F. Rabek (Herausgeber), Elsevier Appl. Sci., London, Ney York 1993, 396f.).

Darüber hinaus können die erfindungsgemäßen Acrylphosphonsäuremonoester oder ihre Mischungen mit anderen radikalisch polymerisierbaren Comonomeren zur Verbesserung der mechanischen Eigenschaften mit organischen oder anorganischen Partikeln oder Fasern gefüllt werden. Bevorzugte anorganische partikuläre Füllstoffe sind amorphe kugelförmige Materialien auf der Basis von Mischoxiden aus SiO₂, ZrO₂ und/oder TiO₂, mikrofeine Füllstoffe, wie pyrogene Kieselsäure oder Fällungskieselsäure, sowie Makro- oder Minifüllstoffe, wie Quarz-, Glaskeramik- oder Glaspulver mit einer durchschnittlichen Teilchengröße von 0,01 bis 5 µm. Weiterhin können auch röntgenopake Füllstoffe, wie Ytterbiumtrifluorid, oder Glasfasern, Polyamid- oder Kohlenstoff-Fasern eingesetzt werden.

Den Acrylphosphonsäuremonoestern oder Mischungen davon können im Bedarfsfall weitere Komponenten zugesetzt werden, vor allem Lösungsmittel, wie Wasser, Methanol, Ethanol, Isopropanol, Methylethylketon, Aceton, Essigsäureethylester, Dimethylformamid, Dimethylsulfoxid oder Mischungen von diesen, sowie Stabilisatoren, UV-Absorber, Farbstoffe, Pigmente oder Gleitmittel. Zur Verwendung in Dentalmaterialien sind als Lösungsmittel Wasser, Ethanol, Aceton und Essigsäureethylester sowie Mischungen davon bevorzugt.

Die erfindungsgemäßen Acrylphosphonsäuremonoester eignen sich besonders als Bestandteil von Dentalmaterialien, wie Befestigungszemente und Füllungskomposite und vor allem Dentaladhäsive. Solche Materialien zeichnen sich durch eine sehr gute Haftung auf unterschiedlichen Substraten, wie der Zahnhartsubstanz und metallischen Substraten, aus und sind unter feuchten Bedingungen hydrolysestabil.

Bevorzugte erfindungsgemäße Dentalmaterialien enthalten die folgenden Komponenten (a), (b), (c), (d) und/oder (e):
(a) 0,5 bis 99 Gew.-%, bevorzugt 10 bis 80 Gew.-% und besonders bevorzugt 20 bis 50 Gew.-% erfindungsgemäße Acrylphosphonsäuremonoester,
(b) 0,01 bis 5 Gew.-% und bevorzugt 0,1 bis 2,0 Gew.-% radikalischer Initiator,
(c) 0 bis 80 Gew.-%, bevorzugt 0 bis 60 Gew.-% und besonders bevorzugt 0 bis 50 Gew.-% radikalisch polymerisierbare Comonomere,
(d) 0 bis 95 Gew.-%, bevorzugt 0 bis 80 Gew.-% und besonders bevorzugt 0 bis 70 Gew.-% Lösungsmittel, insbesondere Wasser, Ethanol, Aceton, Essigsäureethylester oder Mischungen davon sowie Mischungen von Wasser mit den genannten organischen Lösungsmitteln,
(e) 0 bis 90 Gew.-%, besonders bevorzugt in Abhängigkeit von der Anwendung 0 bis 20 Gew.-% (Adhäsiv), 20 bis 60 Gew.-% (Zement) und 60 bis 85 Gew.-% (Füllungskomposit) Füllstoff.

Gemäß einer besonders bevorzugten Ausführungsform sind die erfindungsgemäßen Dentalmaterialien frei von Acrylphosphonsäuren wie sie beispielsweise in der DE 197 46 708 beschrieben werden.

Die Erfindung wird im folgenden anhand von Beispielen näher erläutert.

### Beispiele

### Beispiel 1

### 2-[4-(Hydroxymethoxyphosphoryl)-2-oxa-butyl]-acrylsäure (1)

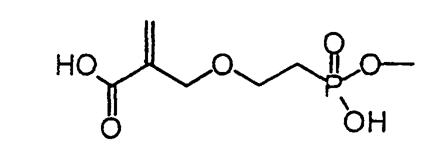

Zu einer Lösung von 120 g (3,0 mol) NaOH in 1200 ml Wasser werden 133 g (0,5mol) 2-[4-(Dimethoxyphosphoryl)-2-oxa-butyl]-acrylsäureethylester, der durch Umsetzung von 2-Hydroxyethylphosphonsäurediethylester mit α-Chlormethylacrylsäureethylester zugänglich ist (N. Moszner, F. Zeuner, U.K. Fischer, V. Rheinberger, Macromol. Chem. Phys. 200 (1999) 1062), unter Eiskühlung so zugetropft, daß die Temperatur 25 °C nicht übersteigt. Dann stellt man die Reaktionsmischung mit ca. 260 ml konz. Salzsäure auf einen pH-Wert von 1 ein. Das Produkt wird 3-mal mit je 500 ml Methylenchlorid gewaschen, die zurückbleibende wäßrige Phase dann mit Natriumchlorid gesättigt und anschließend filtriert. Das Filtrat extrahiert man 3-mal mit je 500 ml Tetrahydrofuran. Nachdem die vereinigten Extrakte über wasserfreiem Na₂SO₄ getrocknet wurden, engt man am Rotationsverdampfer (40 mbar, 50 °C) ein und trocknet den öligen Rückstand im Exsikkator über Phosphorpentoxid bis zur Gewichtskonstanz. Es verbleiben 93,6 g (86 % Ausbeute) eines farblosen Pulvers, daß im Bereich zwischen 71-75 °C schmilzt.

IR (KBr, cm⁻¹): 672 (m), 751 (m), 780 (m), 825 (s), 911 (m), 946 (m), 969 (s), 998 (s), 1014 (s), 1027 (s), 1044 (s), 1055 (s), 1063 (s), 1124 (s), 1188 (m), 1210 (m), 1311 (m), 1372 (w), 1393 (m), 1428 (w), 1448 (m), 1456 (m), 1487 (w), 1634 (s), 1678 (s), 2598 (w), 2672 (w), 2870 (m) und 2900-3200 (b).

¹H-NMR (400 MHz, DMSO-d₆, ppm): 1,95-2,05 (m, 2H, CH₂P), 3,53-3,65 (m, 5H, OCH₂CH₂), 4,08 (s, 2H, =C-CH₂O), 5,82 und 6,13 (s, 2 x 1H,CH₂=C), 10,5 (b, 2H, OH).

¹³C-NMR (100 MHz, DMSO-d₆, ppm): 26,09 und 27,44 (d; CH₂P), 51,56 (CH₃), 64,76 (OCH₂CH₂), 68, 59 (=C-CH₂O), 125, 26 (CH₂=), 138,20 (C=CH₂), 167,11 (C=O).

³¹P-NMR (161,9 MHz, DMSO-d₆): 26,05.

### Beispiel 2

### Radikalische Homopolymerisation des Monomers (1)

2,24 g (10,0 mmol) Monomer **1** und 2,0 Mol-% Azobis(isobutyronitril), bezogen auf Monomer, wurden in 7,7 ml Dimethylformamid in einem Schlenkgefäß gelöst. Die Monomerlösung wurde durch mehrfach wiederholtes Einfrieren unter Argon und Auftauen unter Feinvakuum entgast und anschließend bei 65 °C unter Argon polymerisiert. Während der Polymerisation nimmt die Viskosität der Ausgangslösung deutlich zu. Nach einer Stunde wird die hochviskose Lösung durch Eingießen in die 10-fache Menge an Tetrahydrofuran ausgefällt und nach Trocknen des farblosen Polymerpulvers bis zur Gewichtskonstanz ein Monomerumsatz von 40,1 % bestimmt.

### Beispiel 3

### Untersuchung der hydrolytischen Stabilität von Monomer 1

Das Monomer 1 wird in einer 1:1-Mischung aus Wasser und Ethanol gelöst und eine 20%-ige Lösung wird bei 37 °C gelagert. Wochenweise wird das ¹H-NMR-Spektrum der Lösung aufgenommen. Im Untersuchungszeitraum von 8 Wochen ergab sich keine Veränderung des Spektrums von Monomer 1, was dessen hydrolytische Stabilität belegt.

### Beispiel 4

### Untersuchung der Schmelzhaftung von Monomer 1

Zur Untersuchung der Schmelzhaftung auf Rinderzahnschmelz wurde ein Adhäsiv folgender Zusammensetzung (Angabe in Masse-%) hergestellt:

| | |
|---|---|
| Monomer **1** | 17,4 % |
| Glycerindimethacrylat | 38,2 % |
| 2-Hydroxyethylmethacrylat | 26,3 % |
| Wasser | 17,3 % |
| Photoinitiator | 0,8 % |

Rinderzähne werden so in Kunststoffzylinder eingebettet, daß sich die Schmelzzone und der Kunststoff in einer Ebene befinden. Nach 15 Sekunden Ätzung mit 37%-iger Phosphorsäure wird gründlich mit Wasser abgespült. Dann wird mit einem Microbrush eine Schicht Adhäsiv obiger Zusammensetzung aufgepinselt, mit dem Luftbläser zur Entfernung des Lösungsmittels kurz verblasen und für 40 Sekunden mit einer Halogenlampe (Astralis 7, Vivadent) belichtet. Auf die Adhäsivschicht polymerisiert man einen Kompositzylinder aus Tetric® Ceram (Vivadent) in zwei Schichten von je 1 bis 2 mm auf. Anschließend werden die Prüfkörper 24 h bei 37 °C in Wasser gelagert und anschließend die Scherhaftfestigkeit bestimmt. Es wird ein Wert von 14,0 MPa ermittelt.

### Beispiel 5

### Untersuchung der Löslichkeit und Säurestärke von Monomer 1

Es wurden die Löslichkeit von Monomer **1** in Wasser und Ethanol bestimmt und der pH-Wert einer 20%-igen Lösung des Monomers in einer 1:1-Mischung aus Ethanol und Wasser gemessen. Die Ergebnisse wurden mit den Werten einer strukturanalogen Phosphonsäure verglichen und sind in Tabelle 1 zusammengestellt.

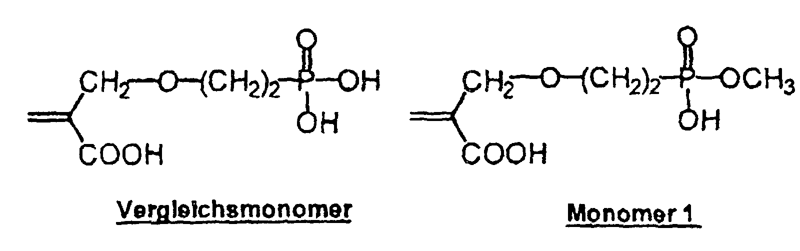

Die Ergebnisse belegen, daß das Monomer **1** in organischen Lösungsmitteln wie Ethanol deutlich besser löslich und stärker acide als das Vergleichsmonomer ist.

Die höhere Acidität des erfindungsgemäßen Monomers äußert sich auch in seinen Ätzvermögen gegenüber Zahnschmelz. So erzeugt eine 40%-ige wäßrige Lösung von Monomer **1** auf Rinderzahnschmelz bereits nach 10 Sekunden ein rasterelktronenmikroskopisch deutlich sichtbares Ätzmuster, während im Fall der entsprechenden Phosphonsäure 30 Sekunden Einwirkungszeit notwendig sind, um die gleiche Ätzwirkung zu erzielen.

Derart deutliche Unterschiede im Lösungsverhalten und in der Ätzwirkung waren bei dem geringen strukturellen Unterschied zwischen Monomer 1 und der Vergleichsverbindung (Austausch einer OCH₃-Gruppe gegen eine OH-Gruppe) nicht zu erwarten.

**Tabelle 1**

| Vergleich der Eigenschaften von Phosphonsäure und Phosphonsäuremonoester | | |
|---|---|---|
| **Parameter** | **Vergleichsmonomer** | **Monomer 1** |
| Wasserlöslichkeit | etwa 40 g/dl | > 50 g/dl |
| Löslichkeit in Ethanol | praktisch unlöslich | > 50 g/dl |
| pH-Wert einer 20%-igen wäßrigen Lösung | 1,25 | 0,61 |

## Patentansprüche

1. Acrylphosphonsäuremonoester der allgemeinen Formel (I), Stereoisomere davon oder Mischungen von diesen 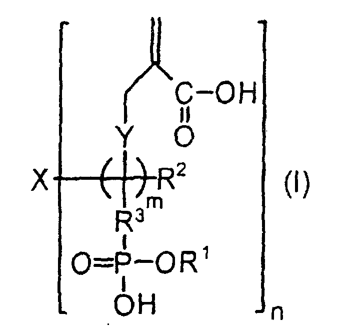 worin R¹, R², R³, X, Y, m und n die folgenden Bedeutungen haben:
R¹ = ein geradkettiger oder verzweigter C₁- bis C₂₀-Alkyl- oder C₆- bis C₁₄-Aryl-Rest;
R² = Wasserstoff, ein geradkettiger oder verzweigter C₁- bis C₅-Alkyl- oder Phenylrest;
R³ = ein geradkettiger oder verzweigter C₁- bis C₈-Alkylenrest, Phenylen oder entfällt;
Y = Sauerstoff, C₁- bis C₈-Alkylen oder entfällt;
m = 0 oder 1;
n = 1 oder 2;
mit der Maßgabe, daß nicht gleichzeitig Y = O, m = 0 und R³ = entfällt bedeuten können und
mit der weiteren Maßgabe daß
für m = 1 und n = 1
X = Wasserstoff oder ein geradkettiger oder verzweigter C₁- bis C₅-Alkylrest oder ein C₆- bis C₁₄-Arylrest;
für m = 1 und n = 2
X = ein geradkettiger oder verzweigter C₁- bis C₁₀-Alkylen-, C₆- bis C₁₀-Arylen-, C₇- bis C₂₀-Arylalkylenrest oder eine chemische Bindung, die zwei Reste mit der in Klammern stehenden Struktur der Formel (I) miteinander verbindet,
wobei die einzelnen Reste substituiert oder unsubstituiert sein können.

2. Acrylphosphonsäuremonoester nach Anspruch 1, **dadurch gekennzeichnet, daß** die Variablen der Formel (I) unabhängig voneinander die folgenden Bedeutungen haben:
R¹ = ein geradkettiger oder verzweigter C₁- bis C₁₀-Alkyl- oder Phenylrest;
R² = Wasserstoff oder ein geradkettiger oder verzweigter C₁- bis C₃-Alkylrest;
R³ = ein geradkettiger oder verzweigter C₁- bis C₄-Alkylenrest, Phenylen oder entfällt;
Y = Sauerstoff oder entfällt; und
X = Wasserstoff oder ein geradkettiger oder verzweigter C₁- bis C₃-Alkylrest (für m = 1 und n = 1); oder
X = ein geradkettiger oder verzweigter C₁- bis C₆-Alkylenrest, Phenylen oder eine chemische Bindung, die zwei Reste mit der in Klammern stehenden Struktur der Formel (I) miteinander verbindet (für m = 1 und n = 2).

3. Acrylphosphonsäuremonoester nach Anspruch 2, **dadurch gekennzeichnet, daß** die Variablen der Formel (I) unabhängig voneinander die folgenden Bedeutungen haben:
R¹ = ein geradkettiger oder verzweigter C₁- bis C₄-Alkylrest, der unsubstituiert oder durch eine OH-Gruppen substituiert sein kann;
R² = Wasserstoff oder ein geradkettiger oder verzweigter C₁- bis C₃-Alkylrest;
R³ = ein geradkettiger oder verzweigter C₁- bis C₄-Alkylenrest, Phenylen oder entfällt;
Y = Sauerstoff oder entfällt.

4. Acrylphosphonsäuremonoester nach einem der Ansprüche 1 bis 3, **dadurch gekennzeichnet, daß** m = 0 oder
für m = 1, n = 2 und
X = Phenylen oder eine chemische Bindung, die zwei Reste mit der in Klammern stehenden Struktur der Formel (I) miteinander verbindet.

5. Verwendung eines Acrylphosphonsäuremonoesters gemäß Anspruch 1 bis 4 als Bestandteil eines Adhäsivs, eines Polymeren, eines Komposits, eines Zements, eines Formkörpers und insbesondere eines Dentalmaterials.

6. Verwendung nach Anspruch 5, **dadurch gekennzeichnet, daß** das Dentalmaterial ein Dentaladhäsiv, ein Befestigungszement oder ein Füllungskomposit ist.

7. Verwendung nach Anspruch 5 oder 6, **dadurch gekennzeichnet, daß** der Acrylphosphonsäuremonoester in zumindest teilweise polymerisierter Form vorliegt.

8. Dentalmaterial, **dadurch gekennzeichnet, daß** es einen Acrylphosphonsäuremonoester gemäß Anspruch 1 bis 4 enthält.

9. Dentalmaterial nach Anspruch 8, **dadurch gekennzeichnet, daß** es den Acrylphosphonsäuremonoester in zumindest teilweise polymerisierter Form enthält.

10. Polymere und Copolymere, **dadurch gekennzeichnet, daß** sie durch Polymerisation oder Copolymerisation eines Acrylphosphonsäuremonoesters gemäß einem der Ansprüche 1 bis 4 erhältlich sind.

## Claims

1. Acrylophosphonic acid monoester of the general formula (I), stereoisomers thereof or mixtures of these 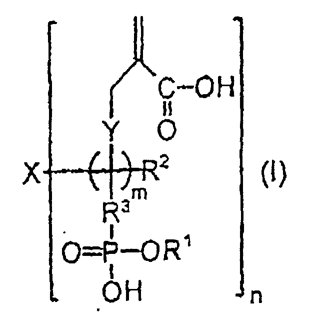 in which R¹, R², R³, X, Y, m and n are as defined below:
R¹ = a straight-chain or branched C₁- to C₂₀-alkyl or C₆- to C₁₄-aryl radical;
R² = hydrogen, a straight-chain or branched C₁- to C₅-alkyl or phenyl radical;
R³ = a straight-chain or branched C₁- to C₈-alkylene radical, phenylene or is absent;
Y = oxygen, C₁- to C₈-alkylene or is absent;
m = 0 or 1;
n = 1 or 2;
with the proviso that Y = 0, m = 0 and R³ = absent cannot be true simultaneously and
with the further proviso that
for m = 1 and n = 1
X = hydrogen or a straight-chain or branched C₁- to C₅-alkyl radical or a C₆- to C₁₄-aryl radical;
for m = 1 and n = 2
X = a straight-chain or branched C₁- to C₁₀-alkylene, C₆- to C₁₀-arylene, C₇- to C₂₀-arylalkylene radical or a chemical bond which bonds two radicals having the bracketed structure of the formula (I) to one another,
where the individual radicals may be substituted or unsubstituted.

2. Acrylophosphonic acid monoester according to Claim 1, **characterized in that** the variables of the formula (I) are, independently of each other, as defined below:
R¹ = a straight-chain or branched C₁- to C₁₀-alkyl or phenyl radical;
R² = hydrogen or a straight-chain or branched C₁- to C₃-alkyl radical;
R³ = a straight-chain or branched C₁- to C₄-alkylene radical, phenylene or is absent;
Y = oxygen or is absent; and
X = hydrogen or a straight-chain or branched C₁- to C₃-alkyl radical (for m = 1 and n = 1); or
X = a straight-chain or branched C₁- to C₆-alkylene radical, phenylene or a chemical bond which bonds two radicals having the bracketed structure of the formula (I) to one another (for m = 1 and n = 2).

3. Acrylophosphonic acid monoester according to Claim 2, **characterized in that** the variables of the formula (I) are, independently of each other, as defined below:
R¹ = a straight-chain or branched C₁- to C₄-alkyl radical, which can be unsubstituted or substituted by an OH group;
R² = hydrogen or a straight-chain or branched C₁- to C₃-alkyl radical;
R³ = a straight-chain or branched C₁- to C₄-alkylene radical, phenylene or is absent;
Y = oxygen or is absent.

4. Acrylophosphonic acid monoester according to one of Claims 1 to 3, **characterized in that** m = 0 or
for m = 1, n = 2 and
X = phenylene or a chemical bond which bonds two radicals having the bracketed structure of the formula (I) to one another.

5. Use of an acrylophosphonic acid monoester according to Claims 1 to 4 as constituent of an adhesive, a polymer, a composite, a cement, a moulding and in particular a dental material.

6. Use according to Claim 5, **characterized in that** the dental material is a dental adhesive, a fixing cement or a filling composite.

7. Use according to Claim 5 or 6, **characterized in that** the acrylophosphonic acid monoester is in at least partially polymerized form.

8. Dental material, **characterized in that** it comprises an acrylophosphonic acid monoester according to Claims 1 to 4.

9. Dental material according to Claim 8, **characterized in that** it comprises the acrylophosphonic acid monoester in at least partially polymerized form.

10. Polymers and copolymers, **characterized in that** they can be obtained by polymerization or copolymerization of an acrylophosphonic acid monoester according to one of Claims 1 to 4.

## Revendications

1. Mono esters de l'acide acrylphosphonique de la formule générale (I), leurs stéréo-isomères ou les mélanges de ces derniers 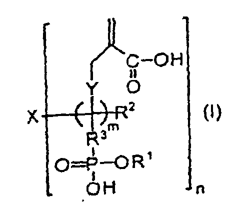 dans lesquels R¹, R², R³, X, Y, m et n ont les significations suivantes :
R¹ : un reste d'alkyle linéaire ou ramifié C₁ à C₂₀ ou un reste d'aryle C₆ à C₁₄ ;
R² : l'hydrogène, un reste d'alkyle linéaire ou ramifié C₁ à C₅ ou un reste de phényle ;
R³ : un reste d'alkylène linéaire ou ramifié C₁ à C₈, du phénylène ou absent;
Y = l'oxygène, l'alkylène C₁ à C₈ ou absent ;
m = 0 ou 1 ;
n = 1 ou 2 ;
étant entendu que Y = 0, m = 0 et R³ = absent et que
également
pour m = 1 et n = 1
X = hydrogène ou un reste d'alkyle linéaire ou ramifié C₁ à C₅ ou un reste d'aryle C₆ à C₁₄ ;
pour m = 1 etn=2
X = un reste d'alkylène linéaire ou ramifié C₁ à C₁₀, un reste d'arylène C₆ à C₁₀, un reste d'arylalkylène C₇ à C₂₀ ou une liaison chimique, qui se lie deux restes avec la structure entre parenthèses de la formule (I),
chaque reste pouvant être substitué ou non substitué.

2. Monoesters de l'acide acrylphosphonique selon la revendication 1, **caractérisés en ce que** les variables de la formule (I) ont indépendamment les unes des autres le significations suivantes :
R¹ : un reste d'alkyle linéaire ou ramifié C₁ à C₁₀ ou un reste de phényle ;
R² : hydrogène ou un reste d'alkyle linéaire ou ramifié C₁ à C₃ ;
R³ : un reste d'alkylène linéaire ou ramifié C₁ à C₄, du phénylène ou absent;
Y = oxygène ou absent ; et
X = hydrogène ou un reste d'alkyle linéaire ou ramifié C₁ à C₃ (pour m = 1 et n = 1) ; ou
X = un reste d'alkylène linéaire ou ramifié C₁ à C₆, du phénylène ou une liaison chimique, qui se lie deux restes avec la structure entre parenthèses de la formule (I) (pour m = 1 et n = 2).

3. Monoesters de l'acide acrylphosphonique selon la revendication 2, **caractérisés en ce que** les variables de la formule (I) ont les significations suivantes, indépendamment les unes des autres:
R¹ : un reste d'alkyle linéaire ou ramifié C₁ à C₄ qui peut être non substitué ou substitué par un groupe OH ;
R² : hydrogène ou un reste d'alkyle linéaire ou ramifié C₁ à C₃ ;
R³ : un reste d'alkylène linéaire ou ramifié C₁ à C₄, du phénylène ou absent;
Y = oxygène ou absent.

4. Monoesters de l'acide acrylphosphonique selon l'une des revendications 1 à 3 , **caractérisés en ce que** m = 0 ou
pour m = 1, n = 2 et
X = du phénylène ou une liaison chimique, qui se lie deux restes avec la structure entre parenthèses de la formule (I).

5. Utilisation d'un monoester de l'acide acrylphosphonique selon les revendications 1 à 4 comme composant d'un adhésif, un polymère, un composite, un ciment, un corps de moulage et notamment un matériau dentaire.

6. Utilisation selon la revendication 5, **caractérisée en ce que** le matériau dentaire est un adhésif dentaire, un ciment de scellement ou un composite de remplissage.

7. Utilisation selon la revendication 5 ou 6, **caractérisée en ce que** le mono ester de l'acide acrylphosphonique se présente sous forme partiellement au moins polymérisée.

8. Matériau dentaire **caractérisé en ce qu'**il contient un mono ester de l'acide acrylphosphonique selon les revendications 1 à 4.

9. Matériau dentaire selon la revendication 8, **caractérisé en ce qu'**il contient le mono ester de l'acide acrylphosphonique sous forme partiellement au moins polymérisée.

10. Polymères et copolymères **caractérisés en ce qu'**ils sont susceptibles d'être obtenus par polymérisation ou copolymérisation d'un monoester de l'acide acrylphosphonique selon l'une des revendications 1 à 4.
